# EUROPEAN PATENT APPLICATION

(11) **EP 4 782 536 A1**
(43) Date of publication of application: **29.07.2026**
(21) Application number: 24867387.3
(22) Date of filing: 14.09.2024
(51) Int. Cl.: C12N 5/10, C07K 16/28, A61K 39/00, A61P 35/00

(54) **GAMMA DELTA T CELL TARGETING PD-L1, AND PREPARATION METHOD THEREFOR AND USE THEREOF**

(30) Priority: 18.09.2023 CN 202311206279
(71) Applicant: Linxcell Biotechnologies, Hefei, Anhui 230601 (CN)
(72) Inventor: CHEN, Long, Beijing 100191 (CN); LIN, Jian, Beijing 100191 (CN); ZHENG, Mengzhu, Beijing 100191 (CN); YANG, Zhanqun, Beijing 100191 (CN)
(74) Representative: Weidner Stern Jeschke
(86) International application number: PCT/CN2024/119072
(87) International publication number: WO 2025/060979

(57) **Abstract**

Provided are a γδT cell targeting PD-L1, and a preparation method therefor and the use thereof. By means of unnatural sugar metabolic labeling, the γδT cell is modified with a first active group, and then same and a PD-L1 targeting group modified with a second active group are subjected to a bioorthogonal reaction, thereby obtaining the γδT cell targeting PD-L1. The γδT cell targeting PD-L1 has a targeted and selective killing effect on PD-L1-positive tumor cells; has the advantages of a short preparation cycle, suitability for large-scale production, uniform and stable quality, etc.; and has good application prospects.

## Description

### TECHNICAL FIELD

The present invention relates to the technical fields of bioengineering and medicine, and specifically to a γδT cell targeting PD-L1, and a preparation method therefor and the use thereof.

### BACKGROUND

The incidence and mortality rates of cancer have been increasing year by year. With the continuous exploration and practice of the body's immunity, immunotherapy-related research has become increasingly popular. Immune cell therapy aiming to activate and enhance the immune system to kill tumors has become the fourth major pillar of cancer treatment. Adoptive immune cell therapies, such as chimeric antigen receptor T (CAR-T) cells, have achieved remarkable results in combating hematologic malignancies, yet they have the disadvantages of high production cost, long preparation time and complicated process.

PD-L1 is usually highly expressed on the surface of tumor cells. As one of the most important immune checkpoints, upon binding to PD-1, PD-L1 can induce apoptosis, anergy and exhaustion of T cells, thereby inhibiting the activation, proliferation and anti-tumor functions of tumor antigen-specific CD8+ T cells and enabling tumor immune escape. Since normal cells also express PD-L1, conventional CAR-T cells cannot kill PD-L1-expressing tumor cells without damaging PD-L1-expressing normal cells.

Therefore, the development of a novel, convenient and low-cost PD-L1 antibody labeling technology for immune cells, which can selectively kill tumor cells without causing damage to normal cells, will play a huge role in promoting immune cell therapy.

### SUMMARY OF THE INVENTION

The objective of the present invention is to provide a γδT cell targeting PD-L1, and a preparation method therefor and the use thereof. The cell has targeted and selective killing effect on PD-L1-positive tumor cells; has the advantages of a short preparation cycle, suitability for large-scale production, uniform and stable quality, etc., thus holding a promising application prospect.

To this end, in a first aspect, the present invention provides a γδT cell targeting PD-L1, which is obtained by a bioorthogonal reaction conjugating a γδT cell modified with a first active group to a PD-L1 targeting group modified with a second active group; the second active group is suitable for undergoing a bioorthogonal reaction with the first active group;

Wherein, the γδT cell modified with the first active group is obtained by metabolically labeling a γδT cell with unnatural sugar molecules; the unnatural sugar molecules comprise at least one selected from Formulas 1 to 6: 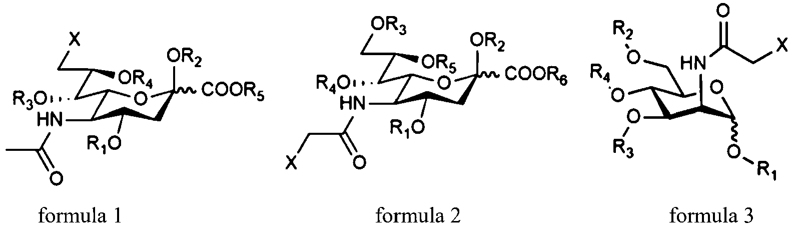 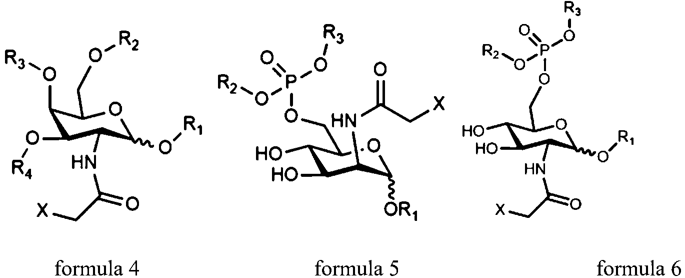
wherein, X is the first active group;
R1, R2, R3, R4, R5 and R6 are each independently selected from hydrogen, halogen, substituted or unsubstituted C_{1~6} alkyl, C_{1~6} alkylthio, C_{1~6} alkylcarbonyl, C_{2~6} alkenyl, C_{2~6} alkynyl and C_{2~6} alkoxy; the said substitution refers to substitution with a group selected from halogen, hydroxyl, amino, nitro, mercapto and C_{1~6} alkyl.

The use of the aforementioned unnatural sugar molecules enables efficient labeling of γδT cells with the first active group, which further facilitates their conjugation to PD-L1 targeting group. Consequently, the γδT cells can target PD-L1-positive tumor cells, possess the ability to distinguish between tumor and normal cells, and significantly enhance the killing effect on tumor cells.

In some embodiments, the unnatural sugar molecules comprise at least one selected from the group consisting of: 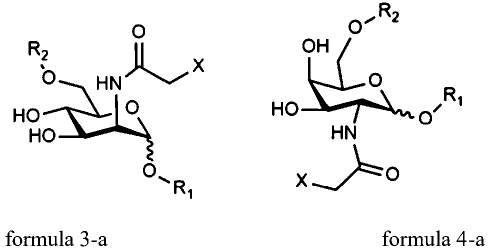
Wherein, X is the first active group;
R1 and R2 are each independently selected from hydrogen, halogen, substituted or unsubstituted C_{1~6} alkyl, C_{1~6} alkylthio, C_{1~6} alkylcarbonyl, C_{2~6} alkenyl, C_{2~6} alkynyl and C_{2~6} alkoxy; the said substitution refers to substitution with a group selected from halogen, hydroxyl, amino, nitro, mercapto and C_{1~6} alkyl.

In some embodiments, the unnatural sugar molecules comprise at least one selected from the group consisting of: 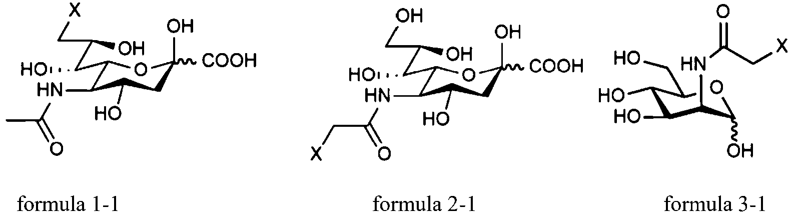 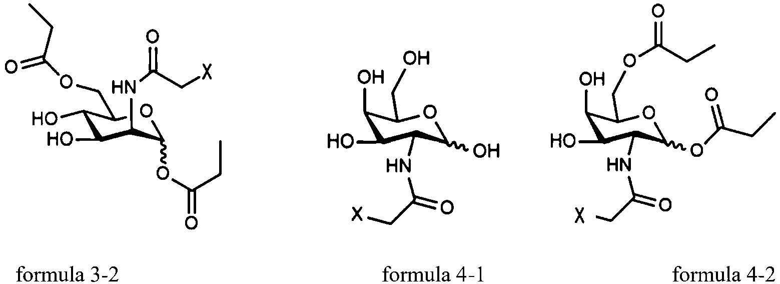 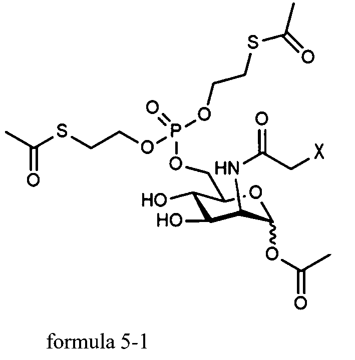 wherein, X is the first active group.

In some embodiments, the bioorthogonal reaction comprises at least one selected from the group consisting of: click chemistry, photocrosslinking, photosensitivity and glycosylation.

In some embodiments, the first active group comprises at least one selected from the group consisting of: azido, alkynyl, trans-cyclooctenyl, triazinyl, tetrazinyl, hydrazido, cyclopropenyl, isocyano and cyclooctynyl.

In some embodiments, the γδT cell is human γδT cell.

In some embodiments, the γδT cell comprises at least one of the following subtypes: Vγ9Vδ2 T cell subtype, Vδ1 T cell subtype.

In some embodiments, the second active group is conjugated to the PD-L1 targeting group via a linker compound; the linker compound has both a group capable of covalent conjugation to a thiol group and the second active group.

In some embodiments, the group capable of covalent conjugation to a thiol group is an N-hydroxysuccinimide active ester group, a maleimide group or a maleic anhydride group.

In some embodiments, the linker compound comprises molecules selected from Formulas L1 and L2: 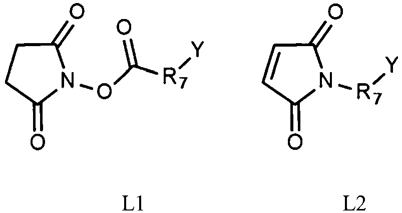
Wherein, Y is the second active group;
R7 is selected from C_{1~20} alkylene or C_{1~20} alkylene ether group.

In some embodiments, R7 is selected from C_{4~20} alkylene or C_{4~20} alkylene ether group.

In some embodiments, the linker compound is DBCO-PEG4-NHS or DBCO-PEG4-MAL;

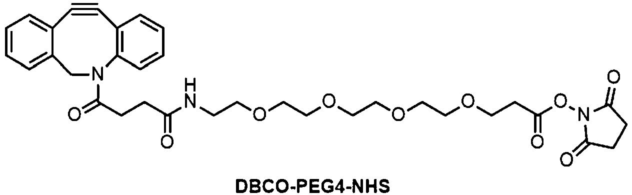

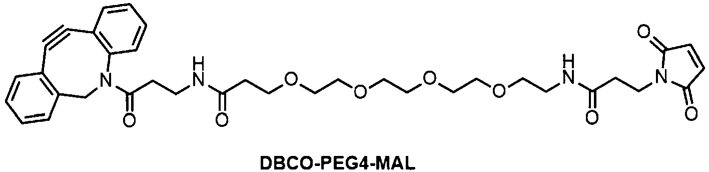

In some embodiments, the PD-L1 targeting group comprises an antibody or its fragment specifically binding to PD-L1.

In some embodiments, the PD-L1 targeting group comprises at least one selected from the group consisting of: immunoglobulin, Fab, Fab', F(ab')2, Fv, scFv, bispecific antibody, polyspecific antibody, single-domain antibody or single-region antibody specifically binding to PD-L1, or monoclonal antibody or polyclonal antibody prepared therefrom.

In a second aspect, the present invention provides a preparation method of the γδT cell targeting PD-L1 described in the first aspect, which comprises: conjugating a γδT cell modified with a first active group to a PD-L1 targeting group modified with a second active group via a bioorthogonal reaction, to obtain the γδT cell targeting PD-L1; the second active group is suitable for undergoing a bioorthogonal reaction with the first active group;

Wherein, the γδT cell modified with the first active group is obtained by metabolically labeling a γδT cell with unnatural sugar molecules; the unnatural sugar molecules comprise at least one selected from Formulas 1 to 6: 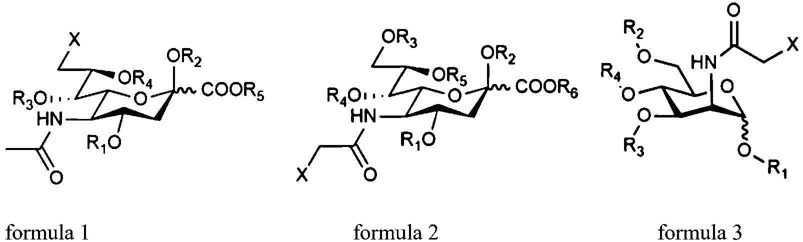 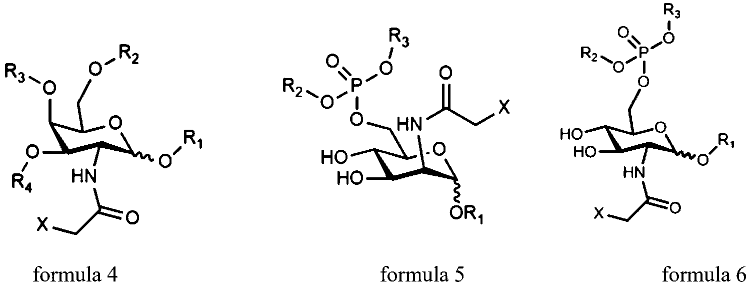
Wherein, X is the first active group;
R1, R2, R3, R4, R5 and R6 are each independently selected from hydrogen, halogen, substituted or unsubstituted C_{1~6} alkyl, C_{1~6} alkylthio, C_{1~6} alkylcarbonyl, C_{2~6} alkenyl, C_{2~6} alkynyl and C_{2~6} alkoxy; the said substitution refers to substitution with a group selected from halogen, hydroxyl, amino, nitro, mercapto and C_{1~6} alkyl.

In some embodiments, the step of metabolic labeling comprises culturing the γδT cells in a medium containing the aforementioned unnatural sugar molecules.

In some embodiments, the concentration of the unnatural sugar molecules in the medium is 10-200 µM; for example, it can be about 10 µM, 25 µM, 50 µM, 75 µM, 100 µM, 125 µM, 150 µM, 175 µM, 200 µM, etc.

In some embodiments, the culture duration is 2-48 h; for example, it can be about 2 h, 6 h, 12 h, 18 h, 24 h, 30 h, 36 h, 42 h, 48 h, etc.

In a third aspect, the present invention provides a use of the γδT cell targeting PD-L1 in preparing products for treating PD-L1-positive tumors.

In some embodiments, the PD-L1-positive tumors comprise at least one selected from the group consisting of: PD-L1-positive lung cancer, lymphoma, intestinal cancer, head and neck squamous cell carcinoma, esophageal cancer, liver cancer, and ovarian cancer.

Compared with the prior art, the technical scheme of the present invention has the following advantages:
(1) The present invention provides a γδT cell targeting PD-L1, and a preparation method therefor. Via the corresponding antibody conjugation method, PD-L1-targeting group can be conveniently and efficiently modified on the surface of γδT cell, to achieve targeted and selective killing of PD-L1-expressing tumor cells.
(2) The γδT cell targeting PD-L1 provided by the present invention has its safety verified at the cellular and animal experimental levels, and can simultaneously improve tumor targeting and enhance the anti-tumor efficacy of γδT cell.

### BRIEF DESCRIPTION OF THE DRAWINGS

A better understanding of other features and advantages of the present invention for those skilled in the art will be obtained by reference to the following detailed description that sets forth preferred embodiments. The drawings are provided only for illustration purposes of the preferred embodiments and are not intended to limit the present invention. In the drawings:
Figure 1 is the schematic flow chart for preparing γδT cells targeting PD-L1;
Figure 2 shows the flow cytometry characterization plots of γδT cell purity in PBMCs before and after culture, with the left plot representing the pre-culture state and the right plot the post-culture state;
Figure 3 is the imaging characterization diagram of γδT cells modified with the first active group; the scale bar is 20 µm;
Figure 4 is the imaging characterization diagram of γδT cells modified with PD-L1-targeting antibodies; the scale bar is 20 µm;
Figure 5 is the in vitro killing assay diagram of γδT-αPDL1 and γδT cells against PD-L1-positive ovarian cancer cells; wherein, _{★★★} indicates P<0.001, _{★★★★} indicates P<0.0001;
Figure 6 is the anti-tumor efficacy evaluation plot in a peritoneal metastasis model of PD-L1-positive ovarian cancer in mice; wherein, _{★★} indicates P<0.01, _{★★★★} indicates P<0.0001.

### DETAILED DESCRIPTION OF THE PREFFERED EMBODIMENTS

In the following detailed description of illustrative embodiments of the present disclosure, reference is made to the accompanying drawings. Although illustrative embodiments of the present disclosure are shown in the accompanying drawings, it should be understood that the present disclosure may be embodied in various forms and is not limited to the embodiments set forth herein. Instead, these embodiments are provided to enable a more thorough understanding of the present disclosure and to fully convey the scope of the present disclosure to those skilled in the art.

In the prior art, immune cell therapy is predominantly based on T lymphocytes. Among them, the T cell receptor (TCR) molecules of the majority (approximately 95%) of mature T cells consist of two heterodimeric peptide chains: α chain and β chain; the TCR molecules of the minority of mature T cells consist of γ chain and δ chain, i.e. γδT cells. γδT cells are innate immune T cells that can kill cancer cells and cancer stem cells, recognize cancer antigens, and exhibit potent cytotoxicity.

In the present invention, a PD-L1 targeting group is conjugated to γδT cells, endowing them with the PD-L1 targeting function while preserving their tumor recognition and killing activities, thereby enabling their enrichment at PD-L1-positive cancer cells and significantly enhancing the cytotoxic effect on tumor cells. To efficiently label γδT cells with PD-L1 targeting group, the present invention provides the unnatural sugar molecules shown in Formulas 1 to 6. Through metabolic labeling, a first active group with bioorthogonal reactivity can be labeled on the surface of yδT cells, which features high labeling efficiency, rapidity and excellent stability.

In some embodiments, the unnatural sugar molecule represented by Formula 5-1-1 is employed.

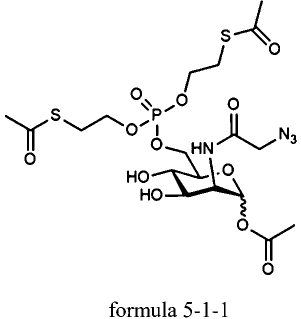

It can be prepared by the following method in two steps:
(1) Synthesis of intermediate 1-O-Ac-ManNAz 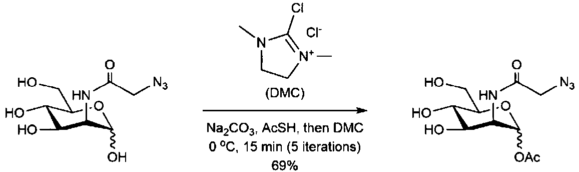 460 mg of ManNAz and 297 mg of sodium carbonate were dissolved in 40 mL of water, and the mixture was cooled to 0°C in an ice-water bath. 195.8 µL of thioacetic acid and 297 mg of DMC (dissolved in 630 µL of water, and the aqueous DMC solution was added during the reaction) were added sequentially. After reacting at 0°C for 15 min, another 297 mg of sodium carbonate and 195.8 µL of thioacetic acid were added, followed by 297 mg of DMC, and the reaction was continued at 0°C for another 15 min. This stepwise feeding procedure was repeated for an additional three rounds (five reaction rounds in total). After the reaction, the reaction system was diluted with 40 mL of water, and the aqueous phase was washed once with DCM and lyophilized to obtain pale yellow powder, which was purified by silica gel column chromatography (dichloromethane and methanol as eluents) to yield 370 mg of 1-O-Ac-ManNAz with a yield of 69%. ¹H NMR (600MHz, Deuterium Oxide) δ 5.96 (dJ = 1.7Hz, 1H), 5.88 (dJ = 1.7Hz, 1H), 4.63 (ddJ = 4.6, 1.8Hz, 1H), 4.48 (ddJ = 4.8, 1.8Hz, 1H), 4.17-4.07 (m, 6H), 3.96 (ddJ = 9.5, 4.5Hz, 1H), 3.91 (ddJ = 12.4, 2.2Hz, 1H), 3.87-3.80 (m, 3H), 3.78 (dddJ = 10.0, 4.1, 2.7Hz, 1H), 3.69 (tJ = 9.9Hz, 1H), 3.61 (tJ = 9.7Hz, 1H), 3.56 (dddJ = 9.9, 4.7, 2.2Hz, 1H), 2.19 (s, 3H), 2.13 (s, 3H).¹³C NMR (151MHz, D₂O) δ 172.03, 171.68, 171.66, 171.03, 92.44, 91.69, 77.20, 74.39, 71.19, 68.67, 66.16, 66.05, 60.05, 60.03, 52.25, 51.66, 51.60, 51.55, 20.22, 20.19. HRMS (ESI) theoretical value C₁₀H₁₆N₄NaO₇[M+Na]⁺327.09167, detection value 327.09094.
(2) Synthesis of Formula 5-1-1: 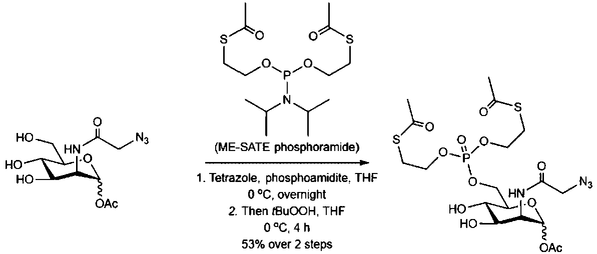 260 mg of 1-O-Ac-ManNAz was dissolved in an appropriate volume of anhydrous tetrahydrofuran to a concentration of 0.08 mol/L. Under a nitrogen atmosphere, three equivalents of Me-SATE phosphonamide (for the synthetic method, see J. Med. Chem. 1995, 38, 3941-3950.) was added to the solution, followed by the addition of four equivalents of 1-H-tetrazine (dissolved in anhydrous acetonitrile at a concentration of approximately 0.45 mol/L) via a syringe. The reaction was carried out at 0°C overnight, and TLC indicated complete consumption of the starting material. Then, 70% tert-butyl hydroperoxide solution was added at 0°C, and the reaction mixture was stirred for 2 h to oxidize the trivalent phosphorus product to the pentavalent phosphorus product. After removing the solvent by rotary evaporation, the crude product was purified by silica gel column chromatography using dichloromethane and methanol as eluents, yielding 260 mg of 1-O-Ac-ManNAz-6-bis-(Me-SATE)-P as the product, with an overall yield of 53% over the two consecutive steps. The compound was characterized by nuclear magnetic resonance (NMR) and high-resolution mass spectrometry (HRMS) as follows. ¹H NMR (600MHz, DMSO-d₆) δ 8.20 (dJ = 7.9Hz, 1.3H), 7.94 (dJ = 9.6Hz, 1.0H), 5.97 (dJ = 1.9Hz, 1.3H), 5.85 (dJ = 1.8Hz, 1.0H), 5.40 (m, 4.6H), 4.54 (dddJ = 9.7, 4.4, 1.8Hz, 1.0H), 4.42 (dddJ = 11.3, 5.9, 1.8Hz, 1.0H), 4.38 (dddJ = 11.4, 6.2, 1.8Hz, 1.3H), 4.28-4.21 (m, 3.6H), 4.21-4.16 (dJ = 5.6Hz, 9.2H), 4.05-3.99 (m, 4.6H), 3.96 (ddJ = 9.4, 4.8Hz, 1.3H), 3.84 (dddJ = 8.8, 6.6, 1.9Hz, 1.3H), 3.80 (ddJ = 9.2, 4.4Hz, 1.0H), 3.69-3.61 (m, 2.3H), 3.49 (tJ = 9.3Hz, 1.0H), 3.28 (m, 9.2H), 2.50 (s, 13.8H), 2.24 (s, 3.9H), 2.14 (s, 3.0H).¹³C NMR (151MHz, DMSO-d₆) δ 194.83, 194.81, 168.78, 168.55, 168.39, 168.36, 92.00, 91.71, 76.22, 76.18, 73.60, 73.56, 70.61, 68.06, 67.46, 67.42, 67.14, 67.10, 66.29, 66.07, 65.76, 65.71, 65.67, 65.66, 65.63, 65.62, 65.60, 51.91, 51.43, 50.77, 50.56, 30.52, 28.82, 28.77, 20.77, 20.69. ³¹P NMR, -1.86. HRMS (ESI) theoretical value C₁₈H₃₀N₄O₁₂PS₂[M+H]⁺589.10393, detection value 589.10428.

Unless otherwise stated, the reagents used in the embodiments of the present invention are commercially available through conventional channels.

### Example 1 Preparation and characterization of γδT cells

### 1. Isolation of Peripheral Blood Mononuclear Cells (PBMCs)

Human peripheral blood was collected, and human PBMCs were isolated using lymphocyte separation medium.

### 2. PBMCs were initially expanded to generate γδT cells

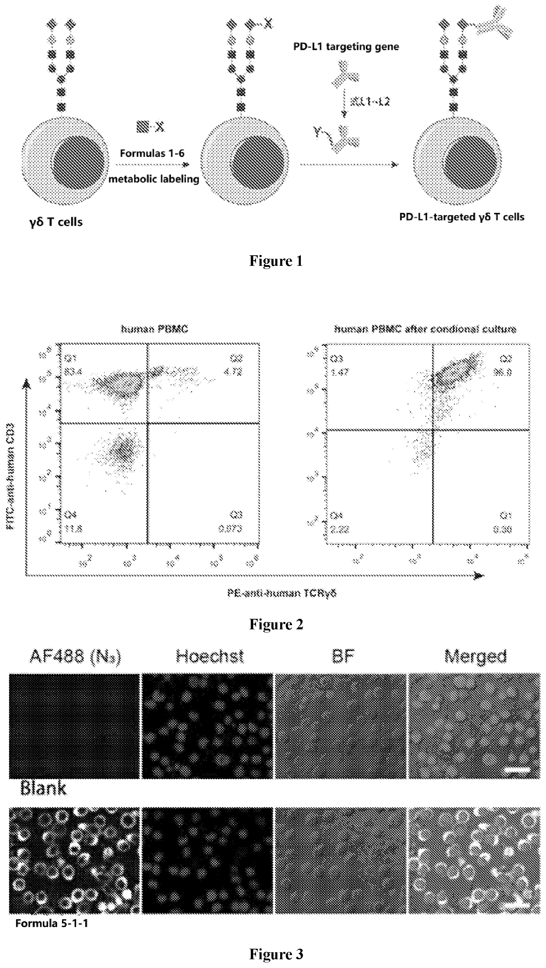

The γδT cells were resuspended at a density of 2×10⁶ cells/mL in the medium described in Table 1, and cultured in a 5% CO₂ incubator at 37°C with static culture for the first three days, followed by regular culture and subculture thereafter. The flow cytometry characterization results of PBMCs before and after culture are shown in Figure 2, which indicated that the purity of γδT cells reached over 95% on the 9th day of culture.

**Table 1. γδT cell activation medium**

| Reagents | 500 mL | 50 mL |
|---|---|---|
| CTS^{™}OpTmizer^{™}T basal medium | 417 mL | 41.7 mL |
| CTS^{™}OpTmizer^{™}T supplemental concentrated medium | 13 mL | 1.3 mL |
| | | |
| IL-2 (5000 IU/mL) | 1 mL | 0.1 mL |
| IL-7 (10 ng/mL) | / | / |
| L-Glutamine 200 mM (100X) | 5 mL | 0.5 mL |
| Aclasta (Zol) (5 µM) | 13.6 mL | 1.36 mL |
| Human autologous plasma (8%)/Human AB serum (10%)/Fetal bovine serum (10%) | 40 mL/50 mL | 4 mL/5 mL |

### Example 2 Preparation and characterization of γδT-αPDL1 cells

### 1 Labeling of azido group on γδT cells

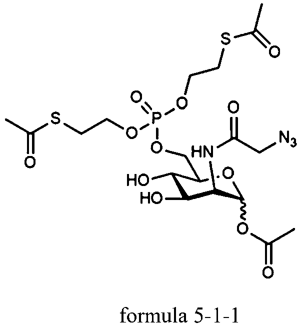

Azido group was used as the first active group to modify γδT cells, and the compound shown in Formula 5-1-1 was employed as the unnatural sugar molecule for the azido group modification. The specific steps are as follows:
The γδT cells identified for purity in Example 1 were collected, and 100 µM unnatural sugar molecules were added for co-cultivation for 24 h. The cells were then harvested by centrifugation at 400 g for 3 min, and washed once with PBS to obtain γδT cells labeled with azido groups (γδT-N₃). A total of 5×10⁴ cells were taken for verification: the cells were co-incubated with 100 µM DBCO-PEG4-Biotin for 10 min at room temperature; after incubation, the cells were washed once with PBS; subsequently, the cells were co-incubated with 2 µg/ml Streptavidin-Alexa Fluor 488 for 15 min at 4°C, followed by one PBS wash. The AF488 fluorescence was detected by laser confocal imaging to characterize the azido group labeling status of the cells. As shown in Figure 3, after the γδT cells were labeled with the unnatural sugar molecules, azido groups were present on the surface of all cells, indicating an extremely high labeling efficiency.

### 2 Labeling of PD-L1 antibody with DBCO

DBCO (dibenzocyclooctyne) can be used for copper-free azide-alkyne cycloaddition reactions. It was modified as the second active group on the PD-L1 antibody, with the specific steps as follows:
The PD-L1 antibody (Alphamab, KN035) was labeled with DBCO-PEG4-NHS at a concentration ratio of 1:5 and reacted at 30°C for 1 h. The reaction system was then passed through a desalting column to remove unreacted DBCO-PEG4-NHS, yielding the PD-L1 antibody labeled with DBCO (designated as DBCO-PD-L1).

### 3. γδT cells targeting PD-L1

The γδT-N₃ cells prepared in Step 1 were co-incubated with the DBCO-PD-L1 prepared in Step 2 for labeling at a DBCO concentration of 20 µM on the antibody surface and a cell density not exceeding 1×10⁷ cells/mL. After labeling at 37°C for 30 min, the cells were harvested by centrifugation at 300 g for 5 min and washed once with PBS, yielding PD-L1-targeting γδT cells, namely γδT-αPDL1 cells.

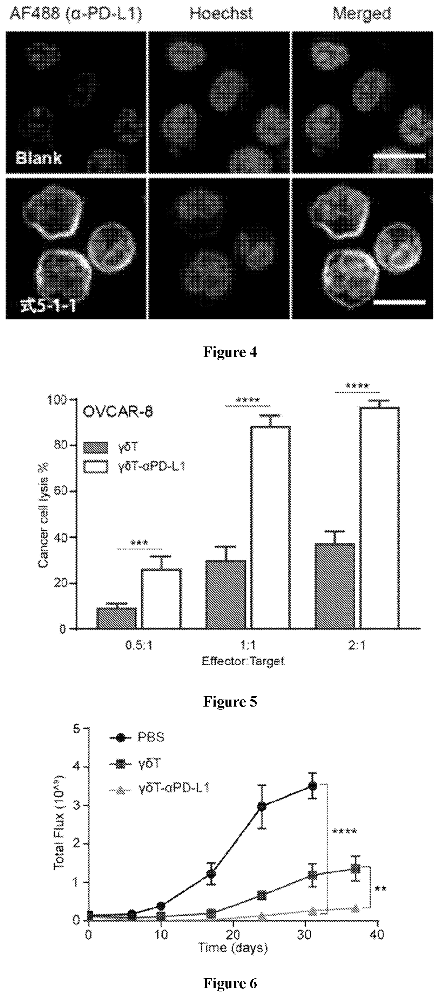

A total of 5×10⁴ cells were taken for verification, and 2 µg/mL recombinant biotinylated PD-L1 protein extracellular domain was added, followed by co-incubation with the cells at room temperature for 15 min. After incubation, the cells were washed once with PBS, then co-incubated with 2 µg/mL Streptavidin-Alexa Fluor 488 at 4°C for 15 min. After incubation, the cells were washed once with PBS again, and the AF488 fluorescence was detected by laser confocal imaging to characterize the PD-L1 antibody labeling status of the cells. The results are shown in Figure 4, demonstrating the presence of anti-PD-L1 antibodies on all cell surfaces with a high labeling efficiency.

### Example 3 Evaluation of in vitro killing efficacy

Ovarian cancer cells OVCAR8 were digested and plated into a 96-well plate at a density of 5000 cells per well, followed by incubation overnight at 37°C with 5% CO₂. Subsequently, γδT-αPDL1 cells and γδT cells were respectively co-incubated with the aforementioned ovarian cancer cells at effector-to-target ratios of 0.5:1, 1:1 and 2:1 for 3 h at 37°C. After incubation, the cell killing efficacy was detected using an LDH assay kit. The results, as shown in Figure 5, indicated that the tumor cell killing efficacy of γδT-αPDL1 cells was significantly superior to that of γδT cells, with a remarkable enhancement in killing efficacy and PD-L1 target specificity.

### Example 4 Evaluation of therapeutic efficacy in a peritoneal metastasis model of ovarian cancer

21 NSG mice aged 6-8 weeks were selected, and 1×10⁶ OVCAR8-LUC ovarian cancer cells were intraperitoneally injected into each mouse. On the second day after the ovarian cancer cell injection, 100 µL of 1 mg/mL D-luciferin potassium salt substrate was intraperitoneally administered, followed by imaging and fluorescence intensity detection. On the third day after the ovarian cancer cell injection, the mice were randomly divided into groups for treatment (7 mice per group): the γδT group was injected with γδT cells, the γδT-αPDL1 group was injected with γδT-αPDL1 cells, and the vehicle control group was injected with PBS. The administration was performed via intraperitoneal injection every 4 days at a dose of 5×10⁶ cells per mouse per time.

Taking the day of first administration as Day 0, imaging was performed, and the fluorescence intensity was detected and statistically analyzed to monitor the changes in tumor volume in mice after treatment. The statistical results of fluorescence intensity are shown in Figure 6, which demonstrated that the administration of γδT-αPDL1 cells resulted in a significant enhancement in the tumor inhibitory effect.

The above descriptions are merely preferred embodiments of the present invention, but the protection scope of the present invention is not limited thereto. Any variation or replacement that can be easily conceived by those skilled in the art within the technical scope disclosed by the present invention shall fall within the protection scope of the present invention. Therefore, the protection scope of the present invention shall be subject to the protection scope of the appended claims.

## Claims

1. A γδT cell targeting PD-L1, **characterized in that** the γδT cell targeting PD-L1 is obtained by a bioorthogonal reaction conjugating a γδT cell modified with a first active group to a PD-L1 targeting group modified with a second active group; the second active group is suitable for undergoing a bioorthogonal reaction with the first active group;
Wherein, the yδT cell modified with the first active group is obtained by metabolically labeling a γδT cell with unnatural sugar molecules; the unnatural sugar molecules comprise at least one selected from Formulas 1 to 6: 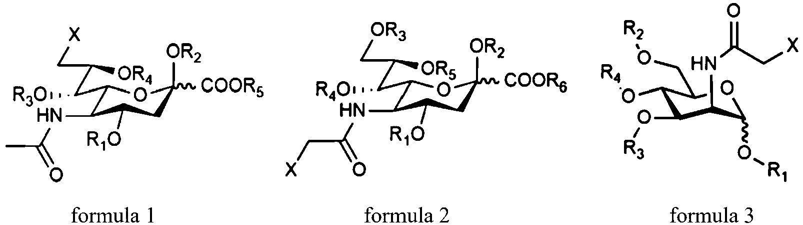 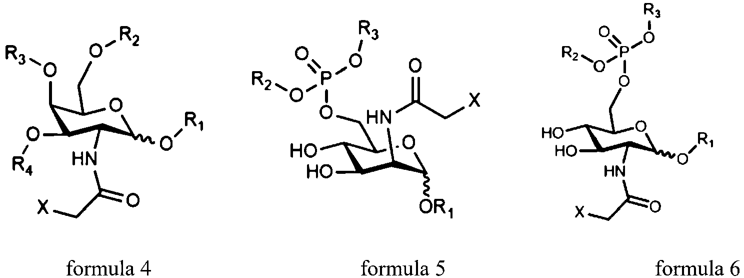
wherein, X is the first active group;
R1, R2, R3, R4, R5 and R6 are each independently selected from hydrogen, halogen, substituted or unsubstituted C_{1~6} alkyl, C_{1~6} alkylthio, C_{1~6} alkylcarbonyl, C_{2~6} alkenyl, C_{2~6} alkynyl and C_{2~6} alkoxy; the said substitution refers to substitution with a group selected from halogen, hydroxyl, amino, nitro, mercapto and C_{1~6} alkyl.

2. The γδT cell targeting PD-L1 of claim 1, **characterized in that** the unnatural sugar molecules comprise at least one selected from the group consisting of: 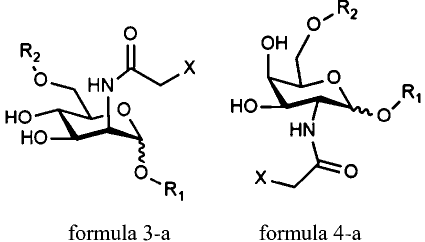
wherein, X is the first active group;
R1 and R2 are each independently selected from hydrogen, halogen, substituted or unsubstituted C_{1~6} alkyl, C_{1~6} alkylthio, C_{1~6} alkylcarbonyl, C_{2~6} alkenyl, C_{2~6} alkynyl and C_{2~6} alkoxy; the said substitution refers to substitution with a group selected from halogen, hydroxyl, amino, nitro, mercapto and C_{1~6} alkyl;
Preferably, the unnatural sugar molecules comprise at least one selected from the group consisting of: 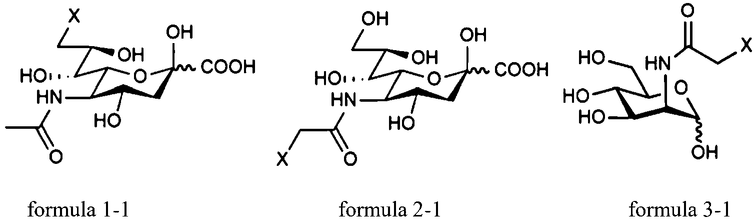 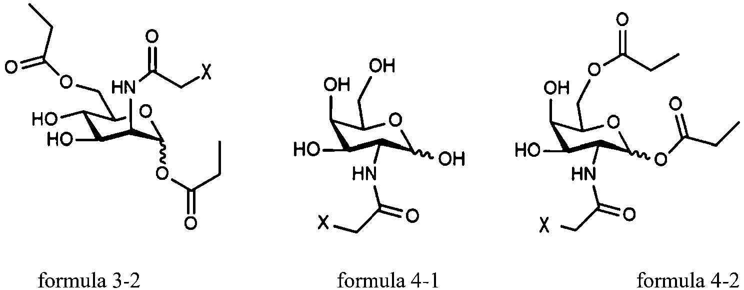 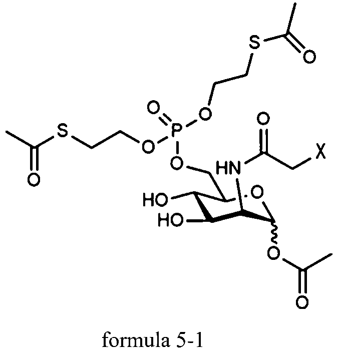
wherein, X is the first active group.

3. The γδT cell targeting PD-L1 of claim 1, **characterized in that** the bioorthogonal reaction comprises at least one selected from the group consisting of: click chemistry, photocrosslinking, photosensitivity and glycosylation;
Preferably, the first active group comprises at least one selected from the group consisting of: azido, alkynyl, trans-cyclooctenyl, triazinyl, tetrazinyl, hydrazido, cyclopropenyl, isocyano and cyclooctynyl.

4. The γδT cell targeting PD-L1 of any one of claims 1-3, **characterized in that** the γδT cell is human γδT cell;
Preferably, the γδT cell comprises at least one of the following subtypes: Vγ9Vδ2 T cell subtype, Vδ1 T cell subtype.

5. The γδT cell targeting PD-L1 of claim 1, **characterized in that** the second active group is conjugated to the PD-L1 targeting group via a linker compound; the linker compound has both a group capable of covalent conjugation to a thiol group and the second active group;
Preferably, the group capable of covalent conjugation to a thiol group is an N-hydroxysuccinimide active ester group, a maleimide group or a maleic anhydride group;
Preferably, the linker compound comprises molecules selected from Formulas L1 and L2: 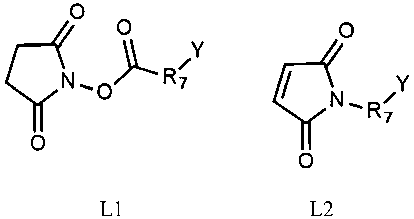
wherein, Y is the second active group;
R7 is selected from C_{1~20} alkylene or C_{1~20} alkylene ether group;
Preferably, the linker compound is DBCO-PEG4-NHS or DBCO-PEG4-MAL.

6. The γδT cell targeting PD-L1 of any one of claims 1-5, **characterized in that** the PD-L1 targeting group comprises an antibody or its fragment specifically binding to PD-L1;
Preferably, the PD-L1 targeting group comprises at least one selected from the group consisting of: immunoglobulin, Fab, Fab', F(ab')2, Fv, scFv, bispecific antibody, polyspecific antibody, single-domain antibody or single-region antibody specifically binding to PD-L1, or monoclonal antibody or polyclonal antibody prepared therefrom.

7. A preparation method of the γδT cell targeting PD-L1 of any one of claims 1-6, **characterized in that** the preparation method comprises: conjugating a γδT cell modified with a first active group to a PD-L1 targeting group modified with a second active group via a bioorthogonal reaction, to obtain the γδT cell targeting PD-L1; the second active group is suitable for undergoing a bioorthogonal reaction with the first active group; 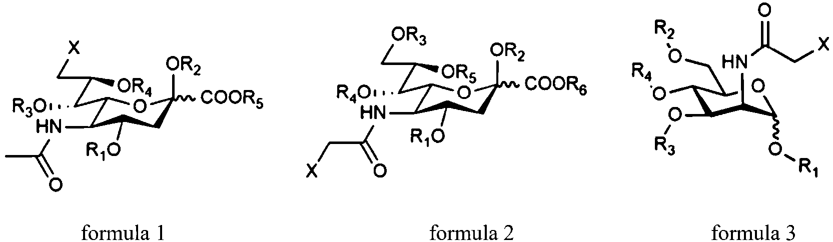 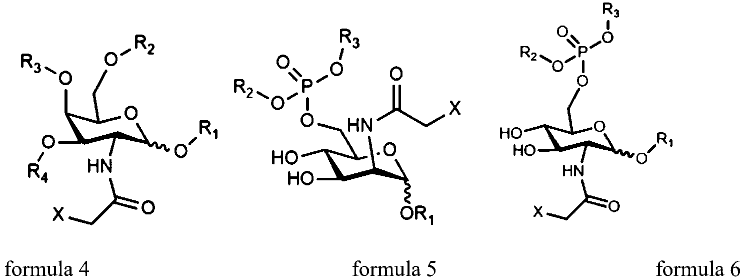 Wherein, the yδT cell modified with the first active group is obtained by metabolically labeling a γδT cell with unnatural sugar molecules; the unnatural sugar molecules comprise at least one selected from Formulas 1 to 6:
Wherein, X is the first active group;
R1, R2, R3, R4, R5 and R6 are each independently selected from hydrogen, halogen, substituted or unsubstituted C_{1~6} alkyl, C_{1~6} alkylthio, C_{1~6} alkylcarbonyl, C_{2~6} alkenyl, C_{2~6} alkynyl and C_{2~6} alkoxy; the said substitution refers to substitution with a group selected from halogen, hydroxyl, amino, nitro, mercapto and C_{1~6} alkyl.

8. The preparation method of claim 7, **characterized in that** the step of metabolic labeling comprises culturing the γδT cells in a medium containing the aforementioned unnatural sugar molecules;
Preferably, the concentration of the unnatural sugar molecules in the medium is 10-200 µM;
Preferably, the culture duration is 2-48 h.

9. A use of the γδT cell targeting PD-L1 in preparing products for treating PD-L1-positive tumors.

10. The use of claim 9, **characterized in that** the PD-L1-positive tumors comprise at least one selected from the group consisting of: PD-L1-positive lung cancer, lymphoma, intestinal cancer, head and neck squamous cell carcinoma, esophageal cancer, liver cancer, and ovarian cancer.
